# FASCICULE DE BREVET EUROPEEN

(11) **EP 3 681 466 B1**
(45) Date de publication et mention de la délivrance du brevet: **20.12.2023**
(21) Numéro de dépôt: 18765125.2
(22) Date de dépôt: 10.09.2018
(51) Int. Cl.: A61K 8/39, A61K 8/60, A61Q 19/00, A61K 8/06, A23L 29/10

(54) **COMBINAISON EMULSIFIANTE POUR L'OBTENTION D'EMULSIONS EAU-DANS-HUILE DE FAIBLE VISCOSITE**
EMULGATORKOMBINATION ZUR HERSTELLUNG VON WASSER-IN-ÖL-EMULSIONEN MIT NIEDRIGER VISKOSITÄT
EMULSIFYING COMBINATION FOR OBTAINING LOW VISCOSITY WATER-IN-OIL EMULSIONS

(30) Priorité: 11.09.2017 FR 1758353
(43) Date de publication de la demande: 22.07.2020
(73) Titulaire: Oleon N.V., 9940 Evergem (Ertvelde) (BE)
(72) Inventeur: PEETERS, Hilde, 3140 Keerbergen (BE)
(74) Mandataire: Santarelli
(86) Numéro de dépôt international: PCT/EP2018/074271
(87) Numéro de publication internationale: WO 2019/048665

(56) Documents cités:
- WO-A1-95/25502
- WO-A1-2013/032318
- WO-A1-2017/104734

## Description

La présente invention concerne la préparation d'une émulsion telle qu'une émulsion eau-dans-huile et plus particulièrement l'utilisation d'un MEL comme co-émulsifiant. La présente invention concerne également une combinaison émulsifiante, une composition et une émulsion la comprenant, ainsi que les procédés de préparation et utilisations afférents.

Une émulsion est une dispersion d'un liquide sous forme de particules ou gouttelettes dans un autre liquide, ces deux liquides étant non miscibles entre eux. Les deux liquides non miscibles d'une émulsion sont généralement désignés par « phase aqueuse » et « phase huileuse ». L'émulsion selon l'invention est plus particulièrement une émulsion eau-dans-huile comportant donc une phase aqueuse dispersée dans une phase huileuse.

Plus particulièrement, les émulsions selon l'invention ont une faible viscosité dynamique à 25°C, c'est-à-dire inférieure à 10 Pa.s, de préférence inférieure à 6 Pa.s. De telles émulsions sont avantageuses pour la préparation de produits pulvérisables tels qu'on peut en trouver dans le domaine de la cosmétique et des produits alimentaires.

Or, de telles émulsions à faible viscosité sont instables.

Pour stabiliser ces émulsions, il est connu d'utiliser des huiles de silicone et/ou des émulsifiants de silicone.

La demande WO2003039508 décrit des émulsions eau-dans-huile fluides et pulvérisables contenant un système d'émulsifiants à base d'un émulsifiant de silicone ayant un HLB inférieur ou égal à 8, un émulsifiant ayant un HLB inférieur à 7 et un émulsifiant ayant un HLB supérieur à 10, la phase lipidique contenant une teneur inférieure à 25% en poids en huile de silicone, préférentiellement cette teneur est comprise entre 2 et 25% en poids, les pourcentages étant exprimés par rapport au poids total de l'émulsion.

La stabilité d'une émulsion eau-dans-huile est d'autant plus difficile à atteindre quand l'émulsion comporte une teneur élevée en phase aqueuse.

La demande US 2002/0102282 A1 décrit l'obtention d'émulsions eau-dans-huile comportant au moins 70% en poids de phase aqueuse par rapport au poids de l'émulsion, en utilisant un émulsifiant choisi par les polyéthers, tels de le polymère PEG-45 (dodecyl glycol) et le copolymère PEG-22 (dodecyl glycol). Ces émulsions ont une viscosité dynamique à 25°C inférieure à 5000 mPa.s.

Toutefois, les substances siliconées et les PEG sont controversés. Les substances siliconées, outre le fait qu'elles seraient très rémanentes, ne sont pas biodégradables, et ont donc un impact négatif sur l'environnement. Les PGE sont suspectés de rendre la peau plus perméable à des corps étrangers qui pourraient potentiellement être dangereux pour la santé humaine.

A ce jour, il existe donc toujours un besoin pour des systèmes émulsifiants de remplacement de ces substances à risque.

En effet, quels que soient les types d'industries dans lesquels ils sont utilisés, il est préférable que les systèmes émulsifiant soient respectueux de l'environnement et non toxiques pour les utilisateurs. Cela est particulièrement important lorsqu'ils sont utilisés dans la préparation d'émulsions cosmétiques, phytosanitaires ou alimentaires.

De plus, il serait intéressant de développer des systèmes émulsifiants ayant en outre une ou plusieurs des caractéristiques suivantes:
- posséder une bonne propriété émulsifiante à faible teneur,
- permettre l'obtention d'émulsions stables,
- permettre l'obtention d'émulsions de faible viscosité,
- permettre l'obtention d'émulsions à taille de particules dispersées réduites, dont l'obtention d'émulsions pourrait s'effectuer selon un procédé réalisable à température ambiante et pression atmosphérique.

La présente divulgation concerne donc l'utilisation d'au moins un lipide de mannosylérythritol (MEL) comme co-émulsifiant d'un polyricinoléate de polyglycérol (PGPR).

Par co-émulsifiant, on entend un composé capable d'optimiser les propriétés de l'émulsion (stabilité..) obtenue avec un émulsifiant.

Par « lipide de mannosylérythritol » ou MEL, on entend une molécule comportant une partie hydrophile formée par le groupe mannosylérythritol, et une partie hydrophobe formée par au moins un groupe acyle.

Par MEL, on désigne plus particulièrement une molécule présentant la formule générale (I) suivante : 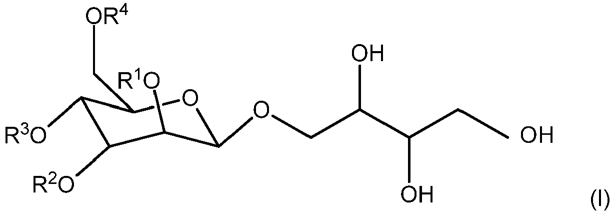 dans laquelle :
- R¹ et R², identiques ou différents, représentent un groupe acyle, comportant une chaine carbonée acyclique insaturée ou saturée,
- R³ et R⁴, identiques ou différents, représentent un groupe acétyle ou un atome d'hydrogène.

Deux stéréoisomères de MEL de formule (I) sont connus et représentés dans les formules (II) et (III) ci-après : 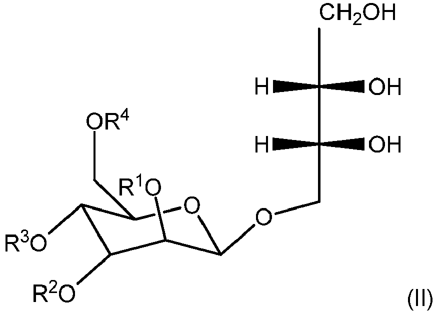 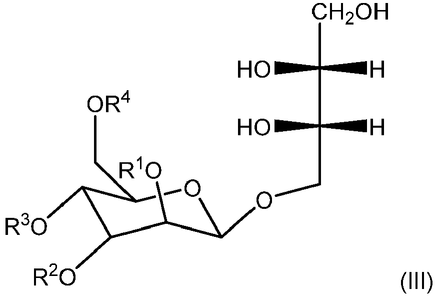 dans lesquelles, R¹, R², R³, R⁴ sont identiques à ceux indiqués en Formule (I).

Les formules (I) à (III) ci-avant peuvent représenter plusieurs molécules, chaque molécule étant donc un MEL. Par « MELs », on désigne au moins deux molécules de formules (I), (II) ou (III) différentes de par leur substitution (groupes acyles, acétyles) ou par leur stéréoisomérie, plus particulièrement, au moins deux molécules de formules (II) différentes.

Par ailleurs, les MELs sont généralement classés en quatre classes de molécules, notées de A à D, selon leur degré d'acétylation en R³ et R⁴. La classe des MELs-A comporte des molécules de formule (I) présentant deux groupes acétyles en R³ et R⁴. La classe des MELs-B et la classe des MELs-C comportent des molécules de formule (I) présentant un seul groupe acétyle en R⁴ et R³ respectivement. Enfin, la classe des MELs-D comporte des molécules de formule (I) ne présentant pas de groupe acétyle (R³= R⁴=H).

Outre de par leur degré d'acétylation, les MELs peuvent varier dans leur structure, de par la nature des acides gras qui composent leur partie hydrophobe. Cette variation est généralement fonction du procédé mis en oeuvre pour l'obtention des MELs.

Les MELs sont généralement obtenus par des procédés mettant en oeuvre la culture de champignons, et plus particulièrement de levures.

Avantageusement, le(s) MEL(s) visé(s) par la présente demande sont obtenus par un procédé de fermentation, comprenant les étapes suivantes :
- la culture d'une souche de champignon et plus particulièrement d'une souche de levure en présence d'une source de carbone pour obtenir des MELs; et
- la récupération des MELs ainsi obtenus.

Les souches à partir desquelles il est possible d'obtenir des MELs sont bien connues de l'homme du métier. A titre d'exemple, il est connu d'utiliser des souches de la famille des Basidiomycètes, de préférence du genre Pseudozyma, telle que *Pseudozyma antarctica, Pseudozyma parantartica, Pseudozyma aphidis, Pseudozyma rugulosa, Pseudozyma graminicola, Pseudozyma siamensis, Pseudozyma hubeiensis, Pseudozyma tsukubaensis, Pseudozyma crassa,* ou du genre Ustilago, telle que *Ustilago maydis, Ustilago cynodontis* et *Ustilago scitaminea.*

En général, selon la souche, une classe de MELs (MELs-A, MELs-B, MELs-C ou MELs-D), est produite majoritairement, voire exclusivement par rapport aux autres classes de MEL. A titre d'exemple, *Pseudozyma antarctica, Pseudozyma aphidis, Pseudozyma rugulosa* et *Pseudozyma parantarctica* produisent en majorité des MELs-A de formule (III). *Pseudozyma graminicola, Pseudozyma siamensis, Pseudozyma hubeiensis* produisent en majorité des MELs-C de formule (III). *Pseudozyma tsukubaensis* produit en majorité des MELs-B de formule (IV) et *Pseudozyma crassa* produit en majorité des MELs- A de formule (IV).

Avantageusement, les MELs sont obtenus par un procédé de fermentation mettant en oeuvre une souche produisant des MELs de formule (II).

Plus particulièrement, les MELs sont obtenus par un procédé de fermentation mettant en oeuvre une souche choisie parmi *Pseudozyma aphidis, Pseudozyma rugulosa, Pseudozyma antarctica* ou *Pseudozyma parantarctica,* préférentiellement parmi *Pseudozyma aphidis, Pseudozyma antarctica* ou *Pseudozyma parantarctica,* plus préférentiellement, la souche est *Pseudozyma aphidis.*

Le substrat carboné est typiquement un glycérol, un n-alcane ou une huile, en particulier d'origine renouvelable.

Toute huile, composée de triglycérides et liquide à la température du procédé de fermentation, peut être utilisée comme substrat carboné.

Préférentiellement, l'huile renouvelable est une huile végétale ou animale, plus préférentiellement, une huile végétale. En particulier, l'huile végétale est choisie parmi le groupe constitué par une huile de soja, une huile de tournesol, une huile d'olive et une huile de colza. Plus particulièrement, l'huile végétale est une huile de soja ou une huile de colza, plus particulièrement encore, une huile de colza.

Ces huiles renouvelables sont particulièrement riches en groupes acyles comportant une chaine carbonée à 18 atomes de carbone, tels que les groupes acyles issus de l'acide oléique, linoléique et/ou linolénique.

Le procédé de fermentation dure généralement au moins 3 jours, préférentiellement au moins 7 jours.

Selon un mode de réalisation préférentiel, les MELs sont obtenus par un procédé de fermentation mettant en oeuvre :
- une souche du genre Pseudozyma, préférentiellement *Pseudozyma antartica, Pseudozyma parantarctica,* ou *Pseudozyma aphidis,*
- une huile végétale, préférentiellement une huile de colza ou une huile de soja, en tant que substrat carboné.

Une telle souche est usuellement cultivée en réacteur dans un milieu comportant du glucose, de l'eau et/ou des sels (tel que le sulfate de magnésium, le phosphate de monopotassium, le nitrate de sodium, et/ou le nitrate d'ammonium). Ce milieu de culture est également mis en oeuvre dans le procédé de fermentation. En effet, d'une manière générale, le milieu de fermentation du procédé de fermentation, comporte un milieu de culture et le substrat carboné.

Avantageusement, les différents composants du milieu (glucose et souche en particulier) sont stérilisés séparément avant introduction dans le réacteur.

La température du milieu est de préférence comprise entre 20°C et 40°C, plus préférentiellement entre 25°C et 35°C. On notera que dans le cadre de la présente demande, et sauf stipulation contraire, les gammes de valeurs indiquées s'entendent bornes incluses.

Le brut réactionnel obtenu à l'issue du procédé de fermentation, est ce qui est appelé dans la présente demande, le brut de fermentation.

Le brut de fermentation comporte généralement au moins deux MELs, au moins du substrat carboné résiduel et/ou un sous-produit du substrat carboné, la souche et de l'eau, le sous-produit du substrat carboné résultant de la fermentation.

L'étape de récupération des MELs a pour objectif de séparer un/des MEL(s) d'un ou de plusieurs des autres composants du brut de fermentation, tels que du substrat carboné résiduel et/ou un sous-produit du substrat carboné, une souche, et/ou de l'eau.

Selon le mode de réalisation préférentiel ci-avant, le brut de fermentation comporte au moins deux MELs, au moins un triglycéride et/ou au moins un acide gras, de l'eau et une souche du genre Pseudozyma.

En effet, lorsque le substrat carboné est une huile d'origine renouvelable, un sous-produit du substrat carboné est un acide gras. De plus, une huile végétale étant principalement (plus de 90% en poids) constituée de triglycérides, l'huile végétale résiduelle est donc composée d'au moins un triglycéride.

La séparation d'un ou des MEL(s) d'un ou de plusieurs des autres composants du brut de fermentation peut se faire par toute méthode de séparation connue de l'homme du métier.

Avantageusement, la séparation d'un ou des MEL(s) d'un ou de plusieurs des autres composants peut comprendre une ou plusieurs des méthodes suivantes :
- décantation,
- centrifugation,
- filtration,
- évaporation,
- extraction liquide/liquide,
- passage sur un substrat minéral ou une résine.

En particulier :
- la souche peut être séparée par décantation, filtration, et/ou centrifugation ;
- l'eau peut être séparée par décantation, évaporation, centrifugation, et/ou passage sur un substrat minéral qui est un adsorbant;
- les acides gras et les triglycérides peuvent être séparés par extraction liquide/liquide et/ou par passage sur un substrat minéral ou une résine.

Les MELs récupérés peuvent donc comporter :
- au moins un triglycéride et/ou au moins un acide gras, et
- optionnellement, une souche.

Par « acide gras », on entend un acide gras libre et/ou sous forme de sel.

La quantité d'acide(s) gras et/ou de triglycéride(s) présente dans les MELs récupérés peut être comprise entre 0,5 et 60% en poids, de préférence entre 1 et 50% en poids, par rapport au poids total de MELs récupérés.

Avantageusement, le ou les acide(s) gras comporte(nt) une chaine carbonée comportant entre 8 et 24 atomes de carbone, de préférence, entre 8 et 20 atomes de carbone.

Avantageusement, le ou les triglycéride(s) comporte(nt) des groupes acyles dont la chaine carbonée acyclique, saturée ou insaturée, comporte entre 8 et 24 atomes de carbones, de préférence entre 16 et 18 atomes de carbone. Plus particulièrement, la chaine carbonée est linéaire et ne comporte que des atomes de carbone et d'hydrogène, éventuellement substituée par une fonction hydroxyle (OH).

Les MELs récupérés peuvent donc se trouver sous une forme plus ou moins purifiée, c'est-à-dire en mélange avec d'autres composants du milieu de fermentation.

Plus particulièrement, dans la présente demande, et en particulier dans les exemples, lorsque les MELs récupérés, sont en mélange avec au moins un acide gras et/ou au moins un triglycéride, optionnellement de l'eau et/ou une souche, ce mélange est appelé « mélange de MELs».

Un premier mélange de MELs est un brut de fermentation, c'est-à-dire au moins deux MELs avec les autres composants du brut de fermentation.

Le brut de fermentation peut faire l'objet d'une ou plusieurs méthodes de séparation, conduisant à d'autres mélanges de MELs préférés présentant les caractéristiques suivantes :
- une teneur en MELs supérieure ou égale à 30% en poids, préférentiellement supérieure ou égale à 40% en poids, plus préférentiellement supérieure ou égale à 50% en poids ;
- une teneur en autres composants (dont acide(s) gras, triglycéride(s), eau, et/ou souche) inférieure ou égale à 70% en poids, préférentiellement inférieure ou égale à 60% en poids, plus préférentiellement inférieure ou égale à 50% en poids ;
les pourcentages en poids étant donnés par rapport au poids du mélange de MELs.

Plus particulièrement, selon la ou les méthode(s) de séparation telle(s) que décrite(s) ci-avant, des mélanges de MELs plus ou moins concentrés en MELs peuvent être obtenus.

Selon un premier mode de réalisation, le mélange de MELs présente les caractéristiques suivantes :
- une teneur en MELs supérieure ou égale à 55% en poids ;
- une teneur en autres composants (dont acide(s) gras, triglycéride(s), eau, et/ou souche) inférieure ou égale à 45% en poids ;
les pourcentages en poids étant donnés par rapport au poids du mélange de MELs.

Avantageusement, dans ce premier mode de réalisation, la teneur en eau et/ou en souche est inférieure ou égale à 10% en poids, préférentiellement inférieur ou égale à 5% en poids, par rapport au poids du mélange de MELs.

Selon un deuxième mode de réalisation, lequel est particulièrement préféré, le mélange de MELs présente les caractéristiques suivantes :
- une teneur en MELs supérieure ou égale à 90% en poids, préférentiellement supérieure ou égale à 95% en poids, plus préférentiellement supérieure ou égale à 98% en poids ;
- une teneur en autre composants (dont acide(s) gras, triglycéride(s), eau, et/ou souche) inférieure ou égale à 10% en poids, préférentiellement inférieure ou égale à 5% en poids, plus préférentiellement inférieure ou égale à 2% en poids ;
les pourcentages en poids étant donnés par rapport au poids du mélange de MELs.

Avantageusement, dans ce deuxième mode de réalisation, la teneur en eau et/ou en souche est inférieure ou égale à 2% en poids, par rapport au poids du mélange de MELs.

Un tel mélange de MELs peut, par exemple, être obtenu à l'aide d'un procédé de fermentation tel que décrit ci-avant, comprenant plusieurs étapes de séparation telles que décrites ci-avant, ces étapes de séparation incluant préférentiellement une extraction liquide/liquide et/ou un passage sur un substrat minéral.

Le passage sur un substrat minéral peut être une chromatographie, telle qu'une chromatographie d'adsorption sur colonne de silice, réalisée à l'aide de solvants adaptés. De tels solvants sont connus de l'homme du métier.

Des exemples de mélanges de MELs et de leur procédé d'obtention sont également décrits dans la publication suivante : "Downstream processing of mannosylerythritol lipids produced by Pseudozyma aphidis" ; Rau et al.; European Journal of Lipids Science and Technology (2005), 107, 373-380.

Par polyricinoléate de polyglycérol (PGPR), on vise une molécule de PGPR ou un mélange de différentes molécules de PGPR.

En effet, du fait de la multiplicité des fonctions hydroxyles présentes dans le glycérol et le polyglycérol, et selon le procédé d'obtention du PGPR, de nombreux produits de réaction sont susceptibles de se former et il se forme généralement un mélange de plusieurs molécules de PGPR qui peuvent différer les unes des autres par :
- le nombre d'unités de glycérol formant le polyglycérol et l'agencement de ces unités,
- le degré d'estérification (c'est à dire le nombre de fonction(s) hydroxyle(s) estérifiée(s) sur le polyglycérol), et/ou
- le nombre d'unité d'acide ricinoléique formant le polyricinoléate.

De préférence, le PGPR comporte au moins un hydroxyle non estérifié.

De préférence, le PGPR comporte majoritairement (au moins 50% en poids sur le poids total de PGPR) un polyricinoléate de polyglycérol-3 (PG-3-PR) ou un polyricinoléate de polyglycérol-4 (PG-4-PR). Le nombre entier suivant le polyglycérol (ou PG) représente le nombre d'unités de glycérol formant le polyglycérol.

De préférence, le PGPR comporte un nombre d'unité d'acide ricinoléique formant le polyricinoléate (PR) compris entre 2 et 12, plus préférentiellement entre 3 et 10, encore plus préférentiellement entre 4 et 6, en particulier, le nombre d'unité d'acide ricinoléique est de 5.

De préférence, la balance hydrophile-lipophile (HLB) du PGPR est comprise entre 2 et 6.

On entend par HLB (Hydrophilic-Lipophilic Balance) l'équilibre entre la dimension et la force du groupe hydrophile et la dimension et la force du groupe lipophile de l'agent tensioactif. La valeur HLB selon GRIFFIN est définie dans J. Soc. Cosm. Chem. 1954 (volume 5), pages 249-256.

Le PGPR est connu pour être un bon émulsifiant pour réaliser des émulsions eau-dans-huile.

US2010/0112170 décrit l'utilisation de PGPR pour la préparation d'émulsions comestibles. Toutefois, le produit final exemplifié dans cette demande est une pâte qui a une structure plastique, et non une émulsion liquide de faible viscosité.

Utilisé à faible teneur, le PGPR seul ne permet pas de stabiliser une émulsion liquide dans le temps.

En revanche, l'utilisation d'un MEL en tant que co-émulsifiant du PGPR, permet de stabiliser une émulsion eau-dans-huile de faible viscosité. Ceci est probablement du au fait que l'ajout de ce co-émulsifiant permet de diminuer la taille de particules de la phase dispersée (phase aqueuse).

En effet, l'utilisation de MEL est particulièrement avantageuse, car de faibles quantités de MEL et de PGPR sont alors suffisantes pour obtenir une émulsion eau-dans-huile liquide de viscosité dynamique à 25°C inférieure à 10 Pa.s, de préférence inférieure à 6 Pa.s., et stable, c'est-à-dire qu'à température ambiante, le volume de substances hydrophobes pouvant apparaitre à la surface de l'émulsion est inférieur à 2% en volume sur le volume de l'émulsion durant les 14 jours suivant la préparation de l'émulsion.

De plus, l'utilisation de MEL en tant que co-émulsifiant de PGPR a un effet synergique. Utilisé seul, le MEL ne permet pas d'obtenir une émulsion eau-dans-huile. La combinaison PGPR et MEL permet d'obtenir une émulsion eau-dans-huile liquide, de faible viscosité, stable, à une teneur en émulsifiants moindre que la teneur en PGPR nécessaire pour obtenir des résultats comparables.

Plus particulièrement, l'invention concerne donc une combinaison émulsifiante comportant :
- au moins un MEL; et
- au moins un PGPR;
dans laquelle le ratio en poids MEL / PGPR est compris entre 10/90 et 40/60.

Les caractéristiques du MEL(s) et du PGPR sont tels qu'indiquées ci-avant.

De préférence, la combinaison émulsifiante selon l'invention comporte au moins deux MELs.

La combinaison émulsifiante selon l'invention permet d'obtenir une émulsion eau-dans-huile aux propriétés améliorées par rapport à une émulsion obtenue à partir d'un PGPR seul.

En effet, l'utilisation de MEL et de PGPR permet d'obtenir une émulsion eau-dans-huile à taille de particules réduite et de plus grande stabilité qu'une émulsion obtenue avec du PGPR seul.

La combinaison émulsifiante possède une bonne propriété émulsifiante à faible teneur. Une teneur d'au moins 0,1% en poids est suffisante pour former une émulsion eau-dans-huile, préférentiellement, la teneur est d'au moins 0,2%, plus préférentiellement d'au moins 0,3% en poids, les pourcentages en poids étant exprimés par rapport au poids de l'émulsion.

La combinaison émulsifiante permet l'obtention d'une émulsion eau-dans-huile liquide de faible viscosité. Par « faible viscosité », on entend une viscosité dynamique à 25°C inférieure à 10 Pa.s, de préférence inférieure à 6 Pa.s.

En particulier, plus la proportion de MEL augmente, plus la viscosité diminue.

La combinaison émulsifiante permet l'obtention d'une émulsion eau-dans-huile stable. Par « stable », on entend qu'à température ambiante, le volume de substances hydrophobes pouvant apparaitre à la surface de l'émulsion est inférieur à 2% en volume sur le volume de l'émulsion durant les 14 jours suivant la préparation de l'émulsion.

En particulier, 1% en poids de combinaison émulsifiante permet de doubler la durée de stabilité d'une émulsion comparativement à une émulsion identique préparée avec 1% de PGPR.

La combinaison émulsifiante permet l'obtention d'une émulsion eau-dans-huile à fine taille de particules dispersées.

En particulier, le diamètre moyen de l'ensemble des particules représentant 50% du volume des particules (« Dv50 ») est inférieur à 20 µm, préférentiellement inférieur à 15µm, plus préférentiellement inférieur à 12µm.

La combinaison émulsifiante selon l'invention peut être mise en oeuvre à température ambiante et pression atmosphérique, notamment lors de la préparation d'une émulsion eau-dans-huile.

Ces caractéristiques de la combinaison émulsifiante selon l'invention sont plus amplement décrites dans l'Exemple 2 ci-après.

Avantageusement, le MEL et le PGPR sont issus de ressources renouvelables et ne présente pas de toxicité pour l'environnement et pour les utilisateurs.

De préférence, dans la combinaison émulsifiante selon l'invention, le ratio en poids MEL(s) / PGPR est compris entre 15/85 et 35/65.

Avantageusement, le ratio en poids MEL(s) / PGPR est compris entre 20/80 et 30/70. Un tel ratio permet encore d'améliorer la stabilité de l'émulsion eau-dans-huile obtenue avec la combinaison émulsifiante selon l'invention.

Avantageusement, le HLB de la combinaison émulsifiante est compris entre 2 et 6, préférentiellement entre 4 et 6.

Avantageusement, la combinaison comporte au moins 50% en poids, préférentiellement au moins 60% en poids de PGPR par rapport au poids total de la combinaison émulsifiante.

Selon un premier mode de réalisation préféré de la combinaison émulsifiante selon l'invention, celle-ci comporte ou consiste en :
- au moins 10% en poids d'au moins un MEL; et
- au moins 60% en poids de PGPR;
dans laquelle le ratio en poids MEL(s) / PGPR est compris entre 10/90 et 40/60.

Les caractéristiques préférées et avantageuses de la combinaison émulsifiante selon l'invention décrites ci-avant s'appliquent à ce mode de réalisation.

L'invention vise également un procédé de préparation de la combinaison émulsifiante selon l'invention, comportant une étape de mélangeage de MEL(s) et de PGPR, dans lequel le ratio en poids MEL(s) / PGPR est compris entre 10/90 et 40/60.

Avantageusement, le mélangeage est réalisé à une température comprise entre 40 et 60°C, de préférence à 60°C.

Les MEL(s) et PGPR peuvent être chauffés indépendamment l'un de l'autre préalablement au mélangeage ou chauffés lors du mélangeage.

Avantageusement, l'émulsifiant et le co-émulsifiant mis en oeuvre dans le procédé de préparation de la combinaison émulsifiante selon l'invention présentent une ou plusieurs des caractéristiques préférées décrites ci-avant.

La combinaison émulsifiante obtenue est fluide à température ambiante et peut donc facilement être manipulée, notamment pour la préparation de compositions et d'émulsions.

L'invention concerne d'ailleurs également une composition comportant :
- au moins un lipide de mannosylérythritol (MEL) ;
- un polyricinoléate de polyglycérol (PGPR); et
- une substance hydrophobe ;
dans laquelle le ratio en poids MEL(s) / PGPR est compris entre 10/90 et 40/60.

Le Mel et le PGPR sont tels que décrits ci-avant. En particulier, la composition selon l'invention comporte au moins deux MELs.

Par « substance hydrophobe », on vise une susbtance pouvant être présente dans la phase huile d'une émulsion, à l'exception des émulsifiant(s) et co-émulsifiant(s) et notamment de l'émulsifiant et co-émulsifiant mentionnés dans la combinaison émulsifiante selon l'invention.

La substance hydrophobe peut être choisie parmi le groupe constitué par les huiles minérales, les silicones, les acides gras, les alcools gras, les esters (autres que le PGPR), leurs dérivés éthoxylés et leurs mélanges.

Plus particulièrement, l'huile minérale est la paraffine liquide.

Plus particulièrement, la silicone est choisie parmi les diméthicones et la cyclopentasiloxane.

Plus particulièrement, l'acide gras est choisi parmi l'acide stéarique, l'acide cétylique et l'acide cétéarylique.

Plus particulièrement, l'alcool gras est choisi parmi l'alcool stéarique, l'alcool cétylique et l'alcool cétéarylique.

Plus particulièrement, l'ester autre que le PGPR, est choisi parmi le groupe constitué par :
- les monoesters obtenus à partir d'un mono-alcool, tel que le méthanol, l'éthanol, le butanol, l'isopropanol, l'octanol, l'heptanol, l'éthylhaxanol, l'alcool stéarylique, l'alcool isostéarylique, le dodécanol, l'isodécanol, l'isononanol, l'isooctanol, et d'un acide mono-carboxylique, tel que l'acide caprylique, l'acide caprique, l'acide heptanoïque, l'acide palmitique, l'acide myristique, l'acide laurique, l'acide isostéarique et l'acide oléique.
- les diesters obtenus à partir d'un mono-alcool, tel que précédemment décrit, et d'un acide di-carboxylique, tel que l'acide fumarique, l'acide adipique, l'acide succinique et l'acide sébacique ;
- les diesters obtenus à partir du monopropylène glycol et d'un acide monocarboxylique tel que décrit ci-avant ;
- les triesters obtenus à partir de glycérol et d'acide(s) gras, et notamment les triglycérides ;
- les tétraesters obtenus à partir d'un tétra-alcool, tel que le pentaérythritol, et d'un acide mono-carboxylique tel que décrit ci-avant ;
et leurs mélanges.

Plus particulièrement, le triglycéride comporte des groupes acyles dont la chaine hydrocarbonée est linéaire.

Avantageusement, le triglycéride est sous forme d'un mélange de triglycérides, qui comportent des chaines hydrocarbonées linéaires saturées, en particulier, des chaines de 8 à 16 atomes de carbone, plus particulièrement des chaines de 6 à 12 atomes de carbone.

De préférence, le mélange de triglycérides est introduit dans la composition via une substance choisie parmi le groupe constitué par les huiles végétales, les cires végétales, les beurres végétaux, les huiles et graisses animales, une fraction particulière d'une des substances ci-avant, ou leurs mélanges. La substance ou la fraction particulière est constituée d'au moins 90% en poids de triglycérides sur le poids de la substance ou de la fraction particulière considérée. Une fraction particulière d'une des substances ci-avant est obtenue par toute méthode connue de l'homme du métier, en particulier par distillation.

Plus préférentiellement, le triglycéride est introduit dans la composition via une huile végétale, une cire végétale, un beurre végétal, une fraction particulière ceux-ci, ou leurs mélanges. Plus préférentiellement encore, le triglycéride est introduit dans la composition via une huile végétale, ou une fraction particulière d'une huile végétale.

A titre d'exemple d'huile végétale, on peut citer l'huile de coco, l'huile de palmiste, l'huile de palme, l'huile d'olive, l'huile de noix, l'huile de colza ou de candula, l'huile de maïs, l'huile de soja, l'huile de tournesol, l'huile de raisin, l'huile de lin, l'huile de camphre, l'huile de noyau d'abricot, l'huile d'avocat, l'huile de noix de macadamia, et l'huile d'amande douce.

A titre d'exemple de cire végétale, on peut citer la cire de soja.

A titre d'exemple de beurre végétal, on peut citer le beurre de karité, le beurre de cacao, le beurre de mangue, le beurre d'olive et le beurre de kokum.

A titre d'exemple d'huile et de graisse animale, on peut citer l'huile de poisson, et le suif.

De préférence, la substance hydrophobe est choisie parmi les esters, autres que le PGPR, plus préférentiellement parmi le groupe d'esters décrit ci-avant.

En particulier, la substance hydrophobe est choisie parmi le succinate de diisostéaryle, le tétraoctanoate de pentaérythritol, le tétraisostéarate de pentaérythritol, le diheptanoate de monopropylène glycol, le laurate d'éthylhexyle, le laurate d'isoamyle, la triéthylhexanoine et leurs mélanges.

De préférence, la substance hydrophobe est choisie parmi le groupe constitué par huiles minérales, les acides gras, les alcools gras, les esters (autres que le PGPR), leurs dérivés éthoxylés et leurs mélanges.

En effet, dans le cadre d'une application cosmétique, la substance hydrophobe n'est pas une silicone.

Avantageusement, la substance hydrophobe est choisie parmi les substances destinées à être mises en contact avec les parties superficielles du corps animal, et plus particulièrement, humain.

Avantageusement, la substance hydrophobe est comestible.

Avantageusement, la substance hydrophobe est liquide à température ambiante et à pression atmosphérique.

Avantageusement, la composition comporte au moins deux substances hydrophobes.

De préférence, la quantité de PGPR est d'au moins 0,15% en poids par rapport au poids total de la composition.

De préférence, la quantité totale en MEL(s) et PGPR est d'au moins 0,3% en poids par rapport au poids de la composition, plus préférentiellement, d'au moins 0,5%.

Avantageusement, la composition selon l'invention comporte en outre une phase aqueuse comportant au moins 50% en poids d'eau, sur le poids total de phase aqueuse.

De préférence, la phase aqueuse comporte au moins 70% en poids, plus préférentiellement au moins 80% en poids d'eau par rapport au poids total de la phase aqueuse.

De préférence, la quantité en phase aqueuse est comprise entre 10 et 90% en poids, plus préférentiellement entre 15 et 85% en poids, encore plus préférentiellement entre 20 et 80% en poids par rapport au poids total de la composition.

La phase aqueuse peut comporter en outre un alcool.

A titre d'exemples d'alcool, on peut citer le glycérol, l'éthanol, le monopropylène glycol, le sorbitol, le xylitol, le 1,3-butylène glycol, le dipropylèneglycol, l'alcool benzylique, le phénoxyéthanol et l'octyldodécanol.

Avantageusement, la phase aqueuse peut comporter en outre un électrolyte. Les électrolytes sont connus pour stabiliser une émulsion eau-dans-huile.

A titre d'exemples d'électrolytes, on peut citer le sulfate de magnésium (MgSO₄) ou le chlorure de magnésium (MgCl₂).

De préférence, l'électrolyte est choisi parmi les électrolytes divalents, tel que le sulfate de magnésium.

Avantageusement, lorsque la composition comporte la phase aqueuse, celle-ci est sous forme d'émulsion eau-dans-huile.

Avantageusement, la combinaison des MEL(s) et PGPR permet d'obtenir une émulsion comportant une grande quantité d'eau, soit jusqu'à 90% d'eau.

De préférence, la composition sous forme d'émulsion est liquide. En particulier, l'émulsion a une viscosité dynamique à 25°C inférieure à 10 Pa.s, de préférence inférieure à 6 Pa.s.

L'invention concerne également un procédé de préparation d'une composition comportant une étape i) de mise en contact et mélangeage d'au moins un MEL, de PGPR et d'une substance hydrophobe, dans lequel le ratio en poids MEL(s) / PGPR est compris entre 10/90 et 40/60.

Avantageusement, le(s) MEL(s), le PGPR et la substance hydrophobe mis en oeuvre dans le procédé de préparation d'une composition selon l'invention présentent une ou plusieurs des caractéristiques préférées décrites ci-avant.

De préférence, l'étape de mélangeage est réalisée sous agitation.

Le mélangeage peut être réalisé à température ambiante ou alternativement, à une température comprise entre 40 et 60°C, de préférence à 60°C.

Le(s) MEL(s), le PGPR et la substance hydrophobe peuvent être chauffés entre 40 et 60°C, de préférence à 60°C, indépendamment l'un de l'autre, préalablement à leur mise en contact ou chauffés lors du mélangeage.

Selon un premier mode de réalisation, une combinaison émulsifiante est préalablement préparée à la mise en contact avec la substance hydrophobe.

La combinaison émulsifiante peut être chauffée préalablement à la mise en contact avec la substance hydrophobe.

Selon un second mode de réalisation, la substance hydrophobe est d'abord mise en contact avec le(s) MEL(s) et/ou le PGPR.

La substance hydrophobe et le(s) MEL(s) et/ou le PGPR peuvent être préalablement chauffés avant la mise en contact.

L'invention vise également un procédé de préparation d'une émulsion eau-dans-huile comportant les étapes suivantes :
i. mise en contact et mélangeage d'au moins un MEL, d'un PGPR et d'une substance hydrophobe pour obtenir une composition
ii. ajout d'eau à la composition obtenue à l'étape i) sous agitation,
dans lequel le ratio en poids MEL(s) / PGPR est compris entre 10/90 et 40/60.

Avantageusement, le(s) MEL(s), le PGPR, la susbstance hydrophobe et l'eau mis en oeuvre dans le procédé de préparation d'une émulsion selon l'invention présente une ou plusieurs des caractéristiques préférées décrites ci-avant.

De préférence, l'agitation à l'étape ii) est réalisée à une vitesse d'au moins 6000 tours/min, plus préférentiellement au moins 8000 tours/min.

L'étape ii) peut nécessiter un chauffage, à adapter selon la température de fusion des ingrédients de la composition.

L'étape i) est telle que décrite ci-avant (caractéristiques avantageuses et préférées et modes de réalisation).

Les combinaisons émulsifiante et compositions selon l'invention et en particulier la composition sous forme d'émulsion eau-dans-huile, peuvent servir à la préparation de produits dans diverses applications.

L'invention concerne également l'utilisation d'une combinaison émulsifiante selon l'invention, ou d'une composition selon l'invention, en particulier sous forme d'émulsion dans des produits cosmétiques, des produits phytopharmaceutiques ou des produits alimentaires.

Une émulsion comprenant la combinaison émulsifiante selon l'invention présente l'avantage de pouvoir être pulvérisée sous forme de fines gouttelettes de diamètre avantageusement inférieur à 20µm. Ainsi, l'émulsion obtenue selon l'invention peut être utilisée dans des produits pulvérisables ou des lingettes imprégnées.

Une émulsion à fines gouttelettes permet en effet de déposer uniformément celle-ci sur une surface, d'améliorer l'étalement de cette émulsion et permettre aux actifs contenus dans les petites gouttelettes de pénétrer plus facilement la surface sur laquelle elles ont été déposées.

L'invention concerne également un produit cosmétique, phytopharmaceutique ou alimentaire comportant une composition selon l'invention, en particulier une composition sous forme d'émulsion.

Par « produit cosmétique », on vise plus particulièrement les produits solaires ou anti-UV, les produits auto-bronzants, les produits démaquillants, les produits de soin pour les cheveux, les brumes hydratantes, les lingettes déodorantes et les lingettes nettoyantes ou démaquillantes.

Le produit cosmétique selon l'invention peut également comporter un ou plusieurs actif(s) cosmétique(s), parfum(s), pigment(s), colorant(s) et/ou conservateur(s).

Par « produit phytopharmaceutique », on vise plus particulièrement les produits insectifuges, insecticides, fongicides et herbicides.

Le produit phytopharmaceutique selon l'invention peut également comporter un ou plusieurs actif(s) phytopharmaceutique(s), tel qu'un actif insectifuge, un actif insecticide, un actif fongicide, un actif herbicide.

Par « produit alimentaire », on vise plus particulièrement les huiles de cuisson et les margarines à teneur réduite en matière grasse.

Le produit alimentaire selon l'invention peut également comporter un ou plusieurs conservateur(s).

Une émulsion comprenant le(s) Mel et le PGPR présente l'avantage de pouvoir être pulvérisée sous forme de fines gouttelettes de diamètre moyen Dv50 avantageusement inférieur à 20µm, préférentiellement inférieur à 15 µm, plus préférentiellement inférieur à 12 µm. Ainsi, l'émulsion obtenu selon l'invention peut être utilisée dans des produits pulvérisables ou des lingettes imprégnées.

L'invention concerne enfin un produit pulvérisable comportant un réservoir et un système de propulsion d'une émulsion, ladite émulsion comportant une combinaison émulsifiante selon l'invention, ou une composition selon l'invention.

L'invention sera mieux comprise au vu des exemples qui suivent, donnés à titre illustratif.

### Exemple 1 : Matériels et méthodes mis en oeuvre dans les Exemples

Les composés utilisés dans les Exemples qui suivent sont les suivants :
- émulsifiants et co-émulsifiant :
   ∘ le mélange de MELs 1B préparé au point 1 ci-dessous (HLB=9) ;
   ∘ le polyglycéryl-3 polyricinoléate, RADIAMULS POLY 2251K d'Oleon (HLB=3,5), (ci-après désigné « PGPR »)
- substances hydrophobes (phase huileuse):
   ∘ des triglycérides caprique/caprylique, Radia 7104 d'Oleon (ci-après désigné « MCT ») ;
   ∘ le laurate d'isoamyle, Jolee 7750 d'Oleon
   ∘ le diheptanoate de propylène glycol, Jolee 7202 d'Oleon ;
   ∘ l'huile de colza, Lesieur
- substances hydrophiles (phase aqueuse) :
   ∘ de l'eau déminéralisée ;

Le matériel spécifique utilisé dans les Exemples :
- un agitateur mécanique IKA^{®} RW 20 ;
- un homogénéisateur Ultra Turrax^{®} T25 IKA-Labortechnik;
- un viscosimètre Brookfield ;
- un granulomètre Mastersizer 2000 de Malvern.

### 1. Obtention de MELs

Les MELs ont été obtenus par un procédé de fermentation comprenant les étapes suivantes :
- la culture d'une souche de levure telle que *Pseudozyma aphidis* en présence d'huile végétale (colza) pour obtenir les MELs; et
- la récupération des MELs ainsi obtenus.

A l'issue de l'étape de récupération des MELs, un premier mélange de MELs (mélange de MELs 1A) est obtenu, qui présente les caractéristiques suivantes :
- Teneur en MELs: 55% en poids
- Teneur en autres composants : 45% en poids (dont 42% en poids d'acides gras libres et de triglycérides et 3% en poids d'eau et de souche),
les pourcentages en poids étant donnés par rapport au poids total du mélange de MELs obtenu.

Une étape de purification du mélange de MELs 1A a ensuite été réalisée par chromatographie d'adsorption sur colonne de silice, avec utilisation d'un mélange de solvants ayant un gradient de polarité croissant. Un second mélange de MELs (mélange de MELs 1B) a ainsi été obtenu, qui présente les caractéristiques suivantes :
- Teneur en MELs: au moins 98% en poids, par rapport au poids total du mélange de MELs obtenu.

En particulier, chacun des mélanges de MELs 1A et 1B comportent des MELs-A à une teneur de 48% en poids, des MELs-B à une teneur de 24% en poids, des MELs-C à une teneur de 27% en poids, et des MELs-D à une teneur de 1% en poids, les pourcentages en poids étant donnés par rapport au poids de la quantité totale de MELs.

### Exemple 2 : Préparation de combinaisons émulsifiantes selon l'invention

Le mélange de MELs 1B et le PGPR sont chauffés à 60°C et mélangés, selon les proportions indiquées dans le Tableau 1 ci-après, à l'aide de l'agitateur mécanique à une vitesse de 500 tours/min jusqu'à l'obtention d'un mélange homogène. Les différentes combinaisons émulsifiantes préparées sont résumées dans le Tableau 1 suivant.

**Tableau 1 : Combinaisons émulsifiantes selon l'invention**

| | Mélange de MELs 1B (%)* | PGPR (%)* | HLB |
|---|---|---|---|
| Combinaison 1 émulsifiante | 10 | 90 | 4,05 |
| Combinaison 2 émulsifiante | 20 | 80 | 4,60 |
| Combinaison 3 émulsifiante | 30 | 70 | 5,15 |
| Combinaison 4 émulsifiante | 40 | 60 | 5,70 |

| | | | |
|---|---|---|---|
| *Les pourcentages indiqués sont des pourcentages en poids sur le poids total de la combinaison | | | |

A température ambiante et à pression atmosphérique, les combinaisons émulsifiantes 1 à 4 sont liquides.

### Exemple 3 : Préparation de compositions et d'émulsions selon l'invention

### 1. Préparation de compositions

Les compositions et émulsions ont été préparées selon les quantités décrites dans le Tableau 2 ci-dessous. Les modes opératoires sont ceux décrits aux points 1 et 2 ci-après.

**Tableau 2 : Compositions préparées à l'Exemple 3**

| | Emulsifiant | Substance hydrophobe | Phase aqueuse |
|---|---|---|---|
| Composition 1 comparative | 1g PGPR | 30g MCT | 69g eau |
| Composition 1 | 1g Combinaison 1 émulsifiante | 30q MCT | 69g eau |
| Composition 2 | 1g Combinaison 2 émulsifiante | 30g MCT | 69g eau |
| Composition 3 | 1g Combinaison 3 émulsifiante | 30g MCT | 69g eau |
| Composition 4 | 1g Combinaison 4 émulsifiante | 30g MCT | 69g eau |

Une combinaison émulsifiante selon l'invention ou un PGPR, et la ou les substance(s) hydrophobe(s) de la phase huileuse (dans le présent Exemple, le MCT) sont mélangé(s) sous agitation mécanique à température ambiante, soit environ 25°C +/- 5°C, jusqu'à l'obtention d'un mélange homogène pour obtenir respectivement une composition selon l'invention ou une composition comparative.

Ce procédé peut être mis en oeuvre si l'ensemble des substances hydrophobes est liquide à température ambiante et pression atmosphérique. C'est le cas du MCT. Dans le cas contraire, la ou les substance(s) hydrophobe(s) peu(ven)t être chauffée(s) à une température égale ou supérieure à la température de fusion la plus élevée des substances en présence avant d'être mélangée(s) à la combinaison émulsifiante ou au PGPR.

### 2. Préparation de compositions sous forme d'émulsion

Aux compositions préalablement préparées, une phase aqueuse est ajoutée (69 g en 520 secondes) sous une agitation mécanique de 800 tr/min. Une fois l'ajout terminé, la composition est agitée 1 minute à 10000 tr/min à l'aide de l'homogénéisateur afin d'obtenir une composition se présentant sous la forme d'une émulsion eau-dans-huile (ci-après désignée « émulsion » dans les Exemples).

On obtient ainsi les émulsions 1 à 4 correspondant aux compositions 1 à 4 selon l'invention et l'émulsion 1 comparative correspondant à la composition 1 comparative.

### Exemple 4 : Evaluation des propriétés des émulsions préparées à l'Exemple 3

### 1. Viscosité dynamique et aspect

La viscosité dynamique des émulsions 1 à 4 selon l'invention et de l'émulsion 1 comparative a été évaluée un jour après leur préparation, à l'aide du viscosimètre Brookfield à une température de 25°C et à une vitesse de 10 tr/min.

Les résultats sont présentés dans le Tableau 3 ci-dessous.

**Tableau 3 : Viscosité dynamique des émulsions 1 à 4 selon l'invention et de l'émulsion 1 comparative préparées à l'Exemple 3**

| | Viscosité dynamique (mPa.s) |
|---|---|
| Emulsion 1 comparative | 2500 |
| Emulsion 1 | 2270 |
| Emulsion 2 | 2190 |
| Emulsion 3 | 1680 |
| Emulsion 4 | 1560 |

Plus la quantité de MEL augmente, plus la viscosité diminue.

L'aspect des différentes émulsions a été évalué à l'oeil nu. Toutes les émulsions sont des émulsions blanches.

### 2. Stabilité

Des échantillons de 15 mL ont été préparés dans des tubes à essai en verre gradués de 15 mL à partir des émulsions précédemment préparées, et laissés au repos à température ambiante.

La stabilité est observée au cours du temps en mesurant le volume de substance(s) hydrophobe(s) (MCT dans le présent Exemple) qui peut apparaitre au-dessus de l'émulsion. Les résultats obtenus sont rassemblés dans le Tableau 4 ci-dessous.

**Tableau 4 : Stabilité à température ambiante des émulsions 1 à 4 selon l'invention et de l'émulsion 1 comparative préparées à l'Exemple 3**

| | **Volume de substance hydrophobe à la surface (mL)** | | | | | |
|---|---|---|---|---|---|---|
| | **1 jour** | **3 jours** | **2 semaines** | **1 mois** | **2 mois** | **3 mois** |
| Emulsion 1 comparative | 0 | < 0,1 | < 0,4 | 1 | 2 | 3 |
| Emulsion 1 | 0 | < 0,1 | 0,1 | 0,5 | 0,7 | 1,5 |
| Emulsion 2 | 0 | < 0,1 | < 0,1 | < 0,1 | < 0,1 | < 0,1 |
| Emulsion 3 | 0 | < 0,1 | < 0,1 | < 0,1 | < 0,1 | < 0,1 |
| Emulsion 4 | 0 | < 0,1 | < 0,1 | 0,1 | 0,3 | 1,5 |

Les résultats montrent qu'en substituant une quantité de PGPR par du MEL, la stabilité augmente. Les émulsions 2 et 3 selon l'invention comprenant 1% en poids de MELs et de PGPR avec un ratio en poids MEL/PGPR respectivement égal à 20/80 et 30/70, présentent une plus grande stabilité.

### 3. Taille des particules dispersées

La mesure de la taille des particules de la phase aqueuse dispersée dans la phase huileuse est mesurée à l'aide du granulomètre cinq jours après la préparation desdites émulsions
Le diamètre moyen des particules, pondéré en volume, est donné selon la terminologie suivante : « Dv50 » représente le diamètre moyen de l'ensemble des particules représentant 50% du volume des particules.

**Tableau 5 : Taille des particules dispersées dans les émulsions 1 à 4 et l'émulsion 1 comparative**

| | Dv50 (µm) |
|---|---|
| Emulsion 1 comparative | 17,806 |
| Emulsion 1 | 10,309 |
| Emulsion 2 | 6,114 |
| Emulsion 3 | 3,328 |
| Emulsion 4 | 5,109 |

En substituant une quantité de PGPR par une quantité de MEL, la taille des particules de phase aqueuse dispersée diminue.

### Exemple 5 : Effet de la quantité de combinaison émulsifiante

Des émulsions comprenant différentes quantités de la combinaison 2 émulsifiante selon l'invention préparée à l'Exemple 2, du MCT et de l'eau ont été préparées, selon la méthode décrite à l'Exemple 3.

Les différentes émulsions préparées sont résumées dans le Tableau 6 suivant.

**Tableau 6: Emulsions comportant différentes quantités de combinaison 2 émulsifiante selon l'invention**

| | Combinaison 2 émulsifiante (%)* | MCT (%)* | Eau (%)* |
|---|---|---|---|
| Emulsion 2-0,1 | 0,1 | 30,0 | 69,9 |
| Emulsion 2-0,3 | 0,3 | 30,0 | 69,7 |
| Emulsion 2-0,5 | 0,5 | 30,0 | 69,5 |
| Emulsion 2-0,8 | 0,8 | 30,0 | 69,2 |

| | | | |
|---|---|---|---|
| *Les pourcentages indiqués sont des pourcentages en poids sur le poids total de l'émulsion. | | | |

Des mesures de viscosités dynamiques, de stabilité et d'évaluation de l'aspect ont été effectuées, de la même manière que dans l'Exemple 4.

Les résultats sur l'aspect, la viscosité et la stabilité à température ambiante des émulsions ainsi préparées sont indiqués dans le Tableau 7 ci-dessous.

**Tableau 7 : Effet de la quantité de combinaison émulsifiante sur l'émulsion**

| Emulsion | Aspect de l'émulsion | Viscosité dynamique (mPa.s) | Volume de substance hydrophobe à la surface (mL) | |
|---|---|---|---|---|
| | | | 1 jour | 3 jours |
| Emulsion 2-0,1 | Blanche | 2280 | < 0,1 | 2 phases |
| Emulsion 2-0,3 | Blanche | 2220 | 0 | < 0,1 |
| Emulsion 2-0,5 | Blanche | 2340 | 0 | < 0,1 |
| Emulsion 2-0,8 | Blanche | 1750 | 0 | < 0,1 |

Quelle que soit la quantité de combinaison émulsifiante, de 0,1% à 0,8% en poids par rapport au poids de l'émulsion, les émulsions sont blanches et de viscosité dynamique équivalente.

Les résultats montrent que 0,1% en poids d'une combinaison émulsifiante comportant un ratio en poids MELs / PGPR de 20/80, par rapport au poids de l'émulsion, suffit pour obtenir une émulsion. Toutefois, à température ambiante, une telle émulsion est déstabilisée au bout de 3 jours.

Des émulsions plus stables sont obtenues en utilisant au moins 0,3% en poids de combinaison 2 émulsifiante selon l'invention.

### Exemple 6 : Effet de la quantité de phase aqueuse

Des émulsions comprenant différentes quantités de phase aqueuse (de l'eau déminéralisée dans le présent Exemple) et de phase huileuse (MCT dans le présent Exemple) ont été préparées selon la méthode décrite à l'Exemple 3 en utilisant la combinaison 2 émulsifiante selon l'invention préparée dans l'Exemple 2.

Les différentes émulsions préparées sont résumées dans le Tableau 8 suivant.

**Tableau 8 : Emulsions comportant 1% de combinaison 2 émulsifiante et différentes quantités de phase aqueuse et de phase huileuse**

| | Combinaison 2 émulsifiante (%)* | MCT (%)* | Eau (%)* |
|---|---|---|---|
| Emulsion 2-89 | 1 | 10 | 89 |
| Emulsion 2-79 | 1 | 20 | 79 |
| Emulsion 2-69 | 1 | 30 | 69 |
| Emulsion 2-59 | 1 | 40 | 59 |
| Emulsion 2-49 | 1 | 50 | 49 |

| | | | |
|---|---|---|---|
| *Les pourcentages indiqués sont des pourcentages en poids sur le poids total de l'émulsion. | | | |

Des mesures de viscosités dynamiques, de stabilité et d'évaluation de l'aspect ont été effectuées, de la même manière que dans l'Exemple 4.

Les résultats sur l'aspect, la viscosité et la stabilité à température ambiante des émulsions ainsi préparées sont indiqués dans le Tableau 9 ci-dessous.

**Tableau 9 : Effet de la quantité de combinaison émulsifiante sur l'émulsion**

| Emulsion | Aspect de l'émulsion | Viscosité dynamique (mPa.s) | Volume substance hydrophobe à la surface (mL) | |
|---|---|---|---|---|
| | | | 8 jours | 14 jours |
| Emulsion 2-89 | blanche | - | 0 | < 0,1 |
| Emulsion 2-79 | blanche | 5020 | 0 | < 0,1 |
| Emulsion 2-69 | blanche | 2285 | 0 | < 0,1 |
| Emulsion 2-59 | blanche | 580 | 0 | < 0,1 |
| Emulsion 2-49 | blanche | 250 | 0 | < 0,1 |

Plus l'émulsion eau-dans-huile comporte d'eau, plus la viscosité augmente.

A température ambiante et pression atmosphérique, une émulsion comportant une forte teneur en eau dispersée, jusqu'à 89% en poids, reste stable au moins 14 jours

### Exemple 7 : Emulsions cosmétiques

### 1. Préparation des émulsions cosmétiques

Les émulsions ont été préparées selon les quantités, en % en poids par rapport au poids de l'émulsion, décrites dans le Tableau 10 ci-dessous. Le mode opératoire est celui décrit à l'Exemple 3, à l'exception de l'homogénéisation finale qui est, dans le présent exemple, de 2 minutes à 9000 tr/min.

**Tableau 10 : Emulsions cosmétiques**

| | Emulsion A comparative | Emulsion B selon l'invention | Emulsion C selon l'invention |
|---|---|---|---|
| Combinaison 2 émulsifiante | | 1 | |
| Combinaison 3 émulsifiante | | | 1 |
| PGPR | 1 | | |
| Jolee 7750 | 15 | 15 | 15 |
| Radia 7104 | 8 | 8 | 8 |
| Jolee 7202 | 10 | 10 | 10 |
| Eau | 65,3 | 65,3 | 65,3 |
| Conservateur | 0,7 | 0,7 | 0,7 |

### 2. Propriétés des émulsions cosmétiques

Les émulsions A, B et C sont blanches.

Des mesures de viscosités dynamiques, de taille des particules dispersées et de stabilité ont été effectuées, de la même manière que dans l'Exemple 4.

Les résultats concernant la viscosité, la taille des particules dispersées et la stabilité à température ambiante des émulsions ainsi préparées sont indiqués dans le Tableau 11 ci-dessous.

**Tableau 11 : Propriétés des émulsions cosmétiques**

| | Viscosité dynamique (mPa.s) | Dv50 (µm) | Volume de substances hydrophobes à la surface (mL) | | |
|---|---|---|---|---|---|
| | | | 1 jour | 4 jours | 7 jours |
| Emulsion A | 520 | 16,642 | < 0,1 | 1 | 2 |
| Emulsion B | 340 | 2,931 | 0 | < 0,1 | < 0,1 |
| Emulsion C | 440 | 10,121 | 0 | < 0,1 | < 0,1 |

Les émulsions B et C selon l'invention ont une viscosité dynamique à 25°C plus faible que l'émulsion A comparative, plus particulièrement inférieure à 500 mPa.s. Les émulsions B et C selon l'invention ont en outre des diamètres de particules Dv50 inférieurs au diamètre Dv50 de l'émulsion comparative et sont plus stables à température ambiante.

Les émulsions B et C selon l'invention peuvent servir de base à la préparation de produits cosmétiques, en particulier des produits cosmétiques pulvérisables.

### Exemple 8 : Huiles de cuisson

### 1. Préparation d'émulsions à utiliser comme huiles de cuisson

Les émulsions ont été préparées selon les quantités, en % en poids par rapport au poids de l'émulsion, décrites dans le Tableau 12 ci-dessous. Le mode opératoire est celui décrit à l'Exemple 3, à l'exception de l'homogénéisation finale qui est de 1 minute à 9000 tr/min. La température des émulsions est ensuite amenée à 15°C.

**Tableau 12 : Emulsion E selon l'invention et émulsion E' comparative**

| | Emulsion E selon l'invention | Emulsion E' comparative |
|---|---|---|
| Combinaison 2 émulsifiante | 0,6 | |
| PGPR | | 0,6 |
| Radia 5666 | 79,4 | 79,4 |
| Eau | 20 | 20 |

### 2. Propriétés des huiles de cuisson

### 2.1. Aspect, viscosité dynamique, diamètre moyen des particules et stabilité

L'aspect des deux émulsions a été évalué à l'oeil nu. L'émulsion E selon l'invention est blanche alors que l'émulsion E' comparative est jaunâtre.

La viscosité dynamique, le diamètre moyen Dv50 et la stabilité ont été évalués comme décrit à l'Exemple 4.

Les résultats sont rassemblés dans le Tableau 13 ci-dessous.

**Tableau 13 : Propriétés des émulsions E selon l'invention et E' comparative La viscosité dynamique des deux émulsions est comparable.**

| | Viscosité dynamique (mPa.s) | Dv50 (µm) | Volume de substance hydrophobe à la surface (mL) | | |
|---|---|---|---|---|---|
| | | | 1 jour | 2 jours | 6 jours |
| Emulsion E selon l'invention | 130 | 0,361 | 0 | 0 | 0 |
| Emulsion E' comparative | 135 | 7,142 | 0 | 4,5 | 2 phases |

L'utilisation du MEL en complément du PGPR permet d'abaisser le diamètre moyen des particules représentant 50% du volume des particules et de stabiliser l'émulsion eau-dans-huile.

### 2.2. Test anti-éclaboussure

Ce test a pour objectif de déterminer le pourcentage d'éclaboussures formées pendant et après le chauffage des émulsions préparées ci-avant.

Une poêle est chauffée à 300°C. 20 g d'une émulsion à 15°C est placée au milieu de la poêle et un papier filtre préalablement pesé, est placé au-dessus de la poêle à 6 cm de la plaque chauffante. Une fois que les éclaboussures sur le papier filtre cessent, ce dernier est à nouveau pesé. La différence de poids équivaut à la masse d'huile de cuisson qui a été éclaboussée en partant de 20 g d'huile de cuisson.

Le test est répété trois fois. Les taux moyens d'éclaboussures calculés pour les deux émulsions sont donnés dans le Tableau 14 ci-dessous.

**Tableau 14 : Taux d'éclaboussures des émulsions E selon l'invention et E' comparative**

| | Taux d'éclaboussures (%) |
|---|---|
| Emulsion E | 0,36 |
| Emulsion E' comparative | 1,58 |

Les résultats montrent que l'utilisation de la combinaison émulsifiante selon l'invention permet de réduire par environ 5 le taux d'éclaboussures, comparativement à l'utilisation de PGPR seul.

Ceci s'explique du fait que les particules dans l'émulsion selon l'invention sont plus petites et que leur hauteur d'éclaboussure est réduite par rapport aux plus grosses particules présentes dans l'émulsion E' comparative. Les petites particules n'atteignant pas le papier filtre situé à 6 cm de la poêle lors de l'éclaboussure, retombent donc dans la poêle.

## Revendications

1. Combinaison émulsifiante comportant :
- au moins un lipide de mannosylérythritol (MEL), et
- au moins un polyricinoléate de polyglycérol (PGPR),
dans laquelle le ratio en poids MEL / PGPR est compris entre 10/90 et 40/60.

2. Procédé de préparation de la combinaison émulsifiante selon la revendication 1, comportant une étape de mélangeage de MEL(s) et de PGPR, dans lequel le ratio en poids MEL(s) / PGPR est compris entre 10/90 et 40/60.

3. Composition comportant :
- au moins un lipide de mannosylérythritol (MEL),
- un polyricinoléate de polyglycérol (PGPR), et
- une substance hydrophobe,
dans laquelle le ratio en poids MEL(s) / PGPR est compris entre 10/90 et 40/60.

4. Composition selon la revendication 3, comportant en outre une phase aqueuse comportant au moins 50% d'eau.

5. Composition selon la revendication 4, laquelle est sous forme d'émulsion eau-dans-huile.

6. Composition selon la revendication 5, **caractérisée en ce que** l'émulsion est liquide.

7. Procédé de préparation d'une composition comportant une étape i. de mise en contact et mélangeage d'au moins un MEL, de PGPR et d'une substance hydrophobe, dans lequel le ratio en poids MEL(s) / PGPR est compris entre 10/90 et 40/60.

8. Procédé de préparation d'une émulsion eau-dans-huile comportant les étapes suivantes :
i. mise en contact et mélangeage d'au moins un MEL, d'un PGPR et d'une substance hydrophobe pour obtenir une composition
ii.ajout d'eau à la composition obtenue à l'étape i) sous agitation,
dans lequel le ratio en poids MEL(s) / PGPR est compris entre 10/90 et 40/60.

9. Utilisation d'une combinaison émulsifiante selon la revendication 1, ou d'une composition selon l'une quelconque des revendications 3 à 6, dans des produits cosmétiques, des produits phytopharmaceutiques ou des produits alimentaires.

10. Produit pulvérisable comportant un réservoir et un système de propulsion d'une émulsion, ladite émulsion comportant une combinaison émulsifiante selon la revendication 1 ou une composition selon l'une quelconque des revendications 3 à 6.

## Patentansprüche

1. Emulgatorkombination, aufweisend:
- mindestens ein Mannosylerythritollipid (MEL), und
- mindestens ein Polyglycerin-Polyricinoleat (PGPR),
wobei das Gewichtsverhältnis MEL/PGPR zwischen 10/90 und 40/60 liegt.

2. Verfahren zur Herstellung der Emulgatorkombination nach Anspruch 1, aufweisend einen Schritt des Mischens von MEL(s) und PGPR, wobei das Gewichtsverhältnis MEL(s)/PGPR zwischen 10/90 und 40/60 liegt.

3. Kombination, aufweisend:
- mindestens ein Mannosylerythritollipid (MEL),
- ein Polyglycerin-Polyricinoleat (PGPR), und
- eine hydrophobe Substanz,
wobei das Gewichtsverhältnis MEL(s)/PGPR zwischen 10/90 und 40/60 liegt.

4. Zusammensetzung nach Anspruch 3, aufweisend ferner eine wässrige Phase, die mindestens 50 % Wasser aufweist.

5. Zusammensetzung nach Anspruch 4, die in Form einer Wasser-in-Öl-Emulsion vorliegt.

6. Zusammensetzung nach Anspruch 5, **dadurch gekennzeichnet, dass** die Emulsion flüssig ist.

7. Verfahren zur Herstellung einer Zusammensetzung, aufweisend einen Schritt i. des Inkontaktversetzens und Mischens mindestens eines MELs, von PGPR und einer hydrophoben Substanz, wobei das Gewichtsverhältnis MEL(s)/PGPR zwischen 10/90 und 40/60 liegt.

8. Verfahren zur Herstellung einer Wasser-in-Öl-Emulsion, aufweisend die folgenden Schritte:
i. Inkontaktversetzen und Mischen mindestens eines MELs, eines PGPRs und einer hydrophoben Substanz, um eine Zusammensetzung zu erhalten,
ii. Hinzufügen von Wasser zu der in Schritt i) erhaltenen Zusammensetzung unter Rühren,
wobei das Gewichtsverhältnis MEL(s)/PGPR zwischen 10/90 und 40/60 liegt.

9. Verwendung einer Emulgatorkombination nach Anspruch 1 oder einer Zusammensetzung nach einem der Ansprüche 3 bis 6 in kosmetischen Produkten, phytopharmazeutischen Produkten oder Lebensmittelprodukten.

10. Sprühfähiges Produkt, aufweisend einen Vorratsbehälter und ein Antriebssystem einer Emulsion, wobei die Emulsion eine Emulgatorkombination nach Anspruch 1 oder eine Zusammensetzung nach einem der Ansprüche 3 bis 6 aufweist.

## Claims

1. Emulsifying combination comprising:
- at least one mannosylerythritol lipid (MEL), and
- at least one polyglycerol polyricinoleate (PGPR),
wherein the MEL / PGPR weight ratio is comprised between 10/90 and 40/60.

2. Process for preparing the emulsifying combination according to claim 1, comprising a step of mixing MEL(s) and PGPR, wherein the MEL(s) / PGPR weight ratio is comprised between 10/90 and 40/60.

3. Composition comprising:
- at least one mannosylerythritol lipid (MEL),
- a polyglycerol polyricinoleate (PGPR), and
- a hydrophobic substance,
wherein the MEL(s) / PGPR weight ratio is comprised between 10/90 and 40/60.

4. Composition according to claim 3, further comprising an aqueous phase comprising at least 50% water.

5. Composition according to claim 4, which is in water-in-oil emulsion form.

6. Composition according to claim 5, **characterised in that** the emulsion is liquid.

7. A process for preparing a composition comprising a step i. of contacting and mixing at least one MEL, PGPR and a hydrophobic substance, wherein the MEL(s) / PGPR weight ratio is comprised between 10/90 and 40/60.

8. Process for preparing a water-in-oil emulsion comprising the following steps:
i. contacting and mixing at least one MEL, a PGPR and a hydrophobic substance to obtain a composition
ii. adding water to the composition obtained in step i) with stirring,
wherein the MEL(s) / PGPR weight ratio is comprised between 10/90 and 40/60.

9. Use of an emulsifying combination according to claim 1, or of a composition according to any one of claims 3 to 6, in cosmetic products, plant protection products or food products.

10. Sprayable product comprising a reservoir and a system for propelling an emulsion, said emulsion comprising an emulsifying combination according to claim 1, or a composition according to any one of claims 3 to 6.
